# EUROPEAN PATENT SPECIFICATION

(11) **EP 4 637 734 B1**
(45) Date of publication and mention of the grant of the patent: **14.10.2026**
(21) Application number: 23841528.5
(22) Date of filing: 20.12.2023
(51) Int. Cl.: A61K 31/135, A61K 31/44, A61K 31/56, A61K 31/7052, A61K 38/16, A61K 48/00, A61P 11/00

(54) **4-(2-(4-((2,4-DIOXOTHIAZOLIDIN-5-YL)METHYL)PHENOXY) DERIVATIVES AS PPAR-Y AGONISTS AND AUTOTAXIN INHIBITORS FOR USE IN THE TREATMENT OF FIBROSIS**
4-(2-(4-(2,4-DIOXOTHIAZOLIDIN-5-YL)METHYL)PHENOXY)-DERIVATE ALS PPAR-Y-AGONISTEN UND AUTOTAXIN-HEMMER ZUR VERWENDUNG BEI DER BEHANDLUNG VON FIBROSE
DÉRIVÉS DE 4-(2-(4-((2,4-DIOXOTHIAZOLIDIN-5-YL)MÉTHYL)PHÉNOXY) EN TANT QU'AGONISTES DE PPAR-Y ET INHIBITEURS DE L'AUTOTAXINE DESTINÉS À ÊTRE UTILISÉS DANS LE TRAITEMENT DE LA FIBROSE

(30) Priority: 22.12.2022 GR 20220101074
(43) Date of publication of application: 29.10.2025
(73) Proprietor: DRUGTREK Private Capital Company, 265 04 Patras, Achaea (GR)
(72) Inventor: AIDINIS, Vasileios, 16346 Hlioupoli (GR); MATRALIS, Alexios, 174 55 Kalamaki, Attika (GR)
(74) Representative: Sonnenhauser, Thomas Martin
(86) International application number: PCT/GR2023/000064
(87) International publication number: WO 2024/134227

(56) References cited:
- EP-A1- 0 306 228
- WO-A1-2010/040130
- US-A1- 2006 270 634
- D'SOUZA KENNETH ET AL: "Autotaxin Is Regulated by Glucose and Insulin in Adipocytes", vol. 158, no. 4, 16 January 2017 (2017-01-16), US, pages 791 - 803, XP093076520, ISSN: 0013-7227, Retrieved from the Internet <URL:http://academic.oup.com/endo/article-pdf/158/4/791/11160820/en.2017-00035.pdf> DOI: 10.1210/en.2017-00035
- SOHDA T ET AL: "STUDIES ON ANTIDIABETIC AGENTS. SYNTHESIS OF 5-Ú4-(1-METHYLCYCLOHEXYLMETHOXY)-BENZYL3?4)THIAZOLIDINE-2,4-DIONE (ADD-3878) AND ITS DERIVATIVES", CHEMICAL AND PHARMACEUTICAL BULLETIN, PHARMACEUTICAL SOCIETY OF JAPAN, JP, vol. 30, no. 10, 1 January 1982 (1982-01-01), pages 3580 - 3600, XP002046259, ISSN: 0009-2363

## Description

The present invention relates to a pharmaceutical compound or pharmaceutically acceptable salt thereof, for use in the prevention or the treatment of fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD due to simultaneous Autotaxin (ATX) inhibition and PPARγ agonism, having the chemical formula selected from the chemical formulas (A-1), (A-2) and (A-3)

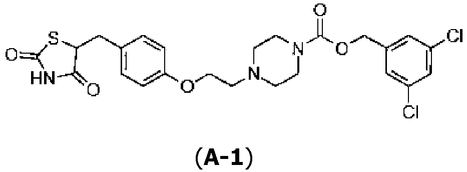

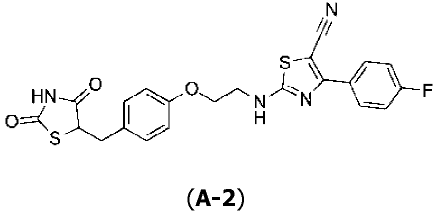

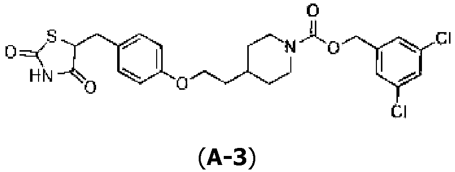

Fibrosis, the exuberant deposition of collagen and other constituents of the extracellular matrix, leads to the modification of tissue architecture, thus disrupting the functional properties of the corresponding organ (lung, liver, skin, kidney, heart, pancreas) and leading to partial or total failure. It is estimated that fibrosis and related fibro-proliferative diseases, including but not limited to pulmonary and hepatic fibrosis, are responsible for ~50% of deaths in developed countries. In addition, the presence of fibrosis decisively affects the spread of cancer and accelerates chronic graft rejection, while the development of fibrosis is a frequent side effect of radiotherapy, but also of COVID19, indicating the enormous impact of fibrosis on human health [Rockey, D. C., P. D. Bell and J. A. Hill (2015). "Fibrosis - A Common Pathway to Organ Injury and Failure." N Engl J Med 372(12): 1138-1149].

Interstitial lung diseases (ILDs) comprise a complex group of pulmonary fibro-proliferative disorders that mainly affect the lung parenchyma, with varied prognosis and clinical behavior. It is estimated that more than 200 separate disorders can cause ILD, including systemic autoimmune diseases such as scleroderma (SSc-ILD) and rheumatoid arthritis (RA-ILD)[Wijsenbeek, M., A. Suzuki and T. M. Maher (2022). "Interstitial lung diseases." The Lancet 400(10354): 769-786]. Idiopathic pulmonary fibrosis (IPF), the most common and most fatal ILD, is a chronic, progressive and usually fatal lung disease. It occurs mainly after the age of 60, while the average survival time from diagnosis ranges from 3-5 years regardless of treatment, a worse prognosis than many types of cancer.

The fibrotic lung shares many features with the aged lung, such as genomic instability, loss of proteostasis, telomere attrition, cellular senescence and dysregulation of mitochondrial homeostasis. Consequently, IPF patients present with significant aging-related comorbidities such as emphysema, lung cancer, pulmonary hypertension, gastroesophageal reflux as well as endocrine/metabolic disorders such as diabetes, hypothyroidism, and dyslipidemia that significantly affect survival and quality of life[Oldham, J. M. and H. R. Collard (2017). "Comorbid Conditions in Idiopathic Pulmonary Fibrosis: Recognition and Management." Front Med (Lausanne) 4: 123]. More specifically, and focusing on metabolic disorders, it has been suggested that hypothyroidism, diabetes and dyslipidemia are prevalent (10-39%) and associated with an adverse prognosis in patients with IPF [Oldham, J. M. and H. R. Collard (2017). "Comorbid Conditions in Idiopathic Pulmonary Fibrosis: Recognition and Management." Front Med (Lausanne) 4: 123]. The metabolic reprogramming involved in IPF, a hallmark of cancer, includes increased glycolysis, glutaminolysis and fatty acid oxidation, leading to dysregulation of mitochondrial homeostasis and function [Selvarajah, B., I. Azuelos, D. Anastasiou and R. C. Chambers (2021). "Fibrometabolism-An emerging therapeutic frontier in pulmonary fibrosis." Sci Signal 14(697)]. Adipokines, cell signaling molecules produced by adipose tissue (e.g. adiponectin, leptin, autotaxin), but also nuclear receptors, such as PPARγ, play a central role in the regulation of metabolic reprogramming, transducing signals from nutritional, hormonal, metabolic and reductive stimuli. Nuclear receptors also regulate the expression of genes involved in cellular processes involved in energy production, including mitochondrial biogenesis and autophagy [Scholtes, C. and V. Giguère (2022). "Transcriptional control of energy metabolism by nuclear receptors." Nature Reviews Molecular Cell Biology 23(11): 750-770].

Autotaxin (ATX) is a secreted lysophospholipase D, widely present in biological fluids, catalysing the extracellular conversion of lysophosphatidylcholine (LPC) to lysophosphatidic acid (LPA), a growth factor-like signalling phospholipid [Barbayianni, E., E. Kaffe, V. Aidinis and G. Kokotos (2015). "Autotaxin, a secreted lysophospholipase D, as a promising therapeutic target in chronic inflammation and cancer." Prog Lipid Res 58: 76-96; Magkrioti, C., A. Galaris, P. Kanellopoulou, E. A. Stylianaki, E. Kaffe and V. Aidinis (2019). "Autotaxin and chronic inflammatory diseases." J Autoimmun 104: 102327] Increased ATX/LPA levels have been reported in different types of cancer in different organs, including the lung [Magkrioti, C., N. Oikonomou, E. Kaffe, M.-A. Mouratis, N. Xylourgidis, I. Barbayianni, P. Megadoukas, V. Harokopos, C. Valavanis, J. Chun, A. Kosma, G. T. Stathopoulos, E. Bouros, D. Bouros, K. Syrigos and V. Aidinis (2018). "The Autotaxin-Lysophosphatidic Acid Axis Promotes Lung Carcinogenesis." Cancer Research 78(13): 3634-3644], the liver [Kaffe, E., A. Katsifa, N. Xylourgidis, I. Ninou, M. Zannikou, V. Harokopos, P. Foka, A. Dimitriadis, K. Evangelou, A. N. Moulas, U. Georgopoulou, V. G. Gorgoulis, G. N. Dalekos and V. Aidinis (2017). "Hepatocyte autotaxin expression promotes liver fibrosis and cancer." Hepatology 65(4): 1369-1383; Kaffe, E., C. Magkrioti and V. Aidinis (2019). "Deregulated Lysophosphatidic Acid Metabolism and Signaling in Liver Cancer." Cancers (Basel) 11(11)] and the pancreas [Auciello, F. R., V. Bulusu, C. Oon, J. Tait-Mulder, M. Berry, S. Bhattacharyya, S. Tumanov, B. L. Allen-Petersen, J. Link, N. D. Kendsersky, E. Vringer, M. Schug, D. Novo, R. F. Hwang, R. M. Evans, C. Nixon, C. Dorrell, J. P. Morton, J. C. Norman, R. C. Sears, J. J. Kamphorst and M. H. Sherman (2019). "A Stromal Lysolipid-Autotaxin Signaling Axis Promotes Pancreatic Tumor Progression." Cancer Discov 9(5): 617-627], as well as chronic inflammatory diseases, including Idiopathic pulmonary fibrosis (IPF) [Oikonomou, N., M. A. Mouratis, A. Tzouvelekis, E. Kaffe, C. Valavanis, G. Vilaras, A. Karameris, G. D. Prestwich, D. Bouros and V. Aidinis (2012). "Pulmonary autotaxin expression contributes to the pathogenesis of pulmonary fibrosis." Am J Respir Cell Mol Biol 47(5): 566-574], different forms of hepatitis [Kaffe, E., A. Katsifa, N. Xylourgidis, I. Ninou, M. Zannikou, V. Harokopos, P. Foka, A. Dimitriadis, K. Evangelou, A. N. Moulas, U. Georgopoulou, V. G. Gorgoulis, G. N. Dalekos and V. Aidinis (2017). "Hepatocyte autotaxin expression promotes liver fibrosis and cancer." Hepatology 65(4): 1369-1383] and rheumatoid arthritis [Nikitopoulou, I., N. Oikonomou, E. Karouzakis, I. Sevastou, N. Nikolaidou-Katsaridou, Z. Zhao, V. Mersinias, M. Armaka, Y. Xu, M. Masu, G. B. Mills, S. Gay, G. Kollias and V. Aidinis (2012). "Autotaxin expression from synovial fibroblasts is essential for the pathogenesis of modeled arthritis." J Exp Med 209(5): 925-933]. Genetic or pharmacologic targeting of ATX attenuated bleomycin (BLM)-induced pulmonary fibrosis, collagen induced arthritis (CIA), CCl4-induced hepatitis, thus establishing ATX as a possible therapeutic target in fibroproliferative diseases and interstitial lung diseases [Magkrioti, C., A. Galaris, P. Kanellopoulou, E. A. Stylianaki, E. Kaffe and V. Aidinis (2019). "Autotaxin and chronic inflammatory diseases." J Autoimmun 104: 102327].

PPARγ is one of 48 existing nuclear receptors that play a central role in regulating metabolic reprogramming by integrating signals from key metabolic sensing systems, such as AMP-activated protein kinase (AMPK) and mechanistic target of rapamycin (mTOR) and synchronizing their activities with the biological clock. Consequently, nuclear receptors are popular multidrug targets for a wide variety of diseases [Scholtes, C. and V. Giguère (2022). "Transcriptional control of energy metabolism by nuclear receptors." Nature Reviews Molecular Cell Biology 23(11): 750-770]. The nuclear receptor and transcription factor PPARγ (peroxisome proliferator-activated receptor regulates the expression of genes involved in the metabolism of lipids and glucose, thereby decisively participating in the maintenance of metabolic homeostasis [Ahmadian, M., J. M. Suh, N. Hah, C. Liddle, A. R. Atkins, M. Downes and R. M. Evans (2013). "PPARγ signaling and metabolism: the good, the bad and the future." Nat Med 19(5): 557-566]. Activators (agonists) of PPARγ include thiazolidinediones (thiazolidinediones, TZD) or glitazones (tro-, pio, rosi-). Diabetes and dyslipidemia are common comorbidities of IPF and are associated with poor prognosis [Oldham, J. M. and H. R. Collard (2017). "Comorbid Conditions in Idiopathic Pulmonary Fibrosis: Recognition and Management." Front Med (Lausanne) 4: 123], while previous studies suggest an association between PPARγ-controlled metabolic disorders and pulmonary fibrosis: TGFβ, the main pro-fibrotic factor, has been shown to suppress PPARγ, while, conversely, PPARγ activation suppressed TGFβ-induced mitochondrial activation [Calvier, L., P. Chouvarine, E. Legchenko, N. Hoffmann, J. Geldner, P. Borchert, D. Jonigk, M. M. Mozes and G. Hansmann (2017). "PPARγ Links BMP2 and TGFβ1 Pathways in Vascular Smooth Muscle Cells, Regulating Cell Proliferation and Glucose Metabolism." Cell Metab 25(5): 1118-1134.e1117]. More recently it has been suggested that the pathogenesis of pulmonary fibrosis involves TGFβ-induced differentiation of lipofibroblasts, a novel subset of pulmonary fibroblasts, into myofibroblasts and that activation of PPARγ inhibits this differentiation, suppressing the formation of pulmonary fibrosis [El Agha, E., A. Moiseenko, V. Kheirollahi, S. De Langhe, S. Crnkovic, G. Kwapiszewska, M. Szibor, D. Kosanovic, F. Schwind, R. T. Schermuly, I. Henneke, B. MacKenzie, J. Quantius, S. Herold, A. Ntokou, K. Ahlbrecht, T. Braun, R. E. Morty, A. Günther, W. Seeger and S. Bellusci (2017). "Two-Way Conversion between Lipogenic and Myogenic Fibroblastic Phenotypes Marks the Progression and Resolution of Lung Fibrosis." Cell Stem Cell 20(2): 261-273.e263]. Genetic deletion of PPARγ exacerbated pulmonary fibrosis in animal models [Malur, A., A. Mohan, R. A. Barrington, N. Leffler, A. Malur, B. Muller-Borer, G. Murray, K. Kew, C. Zhou, J. Russell, J. L. Jones, C. J. Wingard, B. P. Barna and M. J. Thomassen (2019). "Peroxisome Proliferator-activated Receptor-γ Deficiency Exacerbates Fibrotic Response to Mycobacteria Peptide in Murine Sarcoidosis Model." American Journal of Respiratory Cell and Molecular Biology 61(2): 198-208], while pharmacological activation with PPARγ agonists was shown to attenuate BLM-induced pulmonary fibrosis [Kheirollahi, V., R. M. Wasnick, V. Biasin, A. I. Vazquez-Armendariz, X. Chu, A. Moiseenko, A. Weiss, J. Wilhelm, J. S. Zhang, G. Kwapiszewska, S. Herold, R. T. Schermuly, B. Mari, X. Li, W. Seeger, A. Günther, S. Bellusci and E. El Agha (2019). "Metformin induces lipogenic differentiation in myofibroblasts to reverse lung fibrosis." Nat Commun 10(1): 2987], suggesting a beneficial role of PPARγ activation in disease pathogenesis.

Intriguingly, LPA, the enzymatic product of ATX, has been suggested, controversially, to inactivate [D'Souza, K., G. V. Paramel and P. C. Kienesberger (2018). "Lysophosphatidic Acid Signaling in Obesity and Insulin Resistance." Nutrients 10(4)]and/or reduce transcription [Li, L., L. Tam, L. Liu, T. Jin and D. S. Ng (2011). "Wnt-signaling mediates the anti-adipogenic action of lysophosphatidic acid through cross talking with the Rho/Rho associated kinase (ROCK) pathway." Biochem Cell Biol 89(6): 515-521] of PPARγ, although the mechanisms involved, possibly involving activation of signaling by the wnt pathway [Burkhalter, R. J., S. D. Westfall, Y. Liu and M. S. Stack (2015). "Lysophosphatidic Acid Initiates Epithelial to Mesenchymal Transition and Induces β-Catenin-mediated Transcription in Epithelial Ovarian Carcinoma." Journal of Biological Chemistry 290(36): 22143-22154], but also negative regulation of the ATX/LPA axis by PPARγ, remain largely unknown.

Document US9051320B1 relates to a method for preventing or delaying the onset of metabolic diseases through a combination of an autotaxin inhibitor with a glucose-lowering agent. Documents EP3302490B1, US10183949B2, US20170037030A1 and US10125132B2 refer to chemical compounds with autotaxin inhibitory activity. GR1010268B (N-[2-(4-bromophenyl)-2,5-dihydro-4H-thieno[3,4-c]pyrazol-3-yl-acetamides with autotaxin inhibitory activity), GR1010099B (Thieno[3,4-c]pyrazol-3-yl acetamides with autotaxin inhibitory activity) as well as PCT WO2022003377A1 (Thieno[3,4-c]pyrazol-3-yl acetamides as autotaxin inhibitors) are also referred to chemical compounds with autotaxin inhibitory activity.

Documents US2006/270634 A1 and "Kenneth D'Souza, Daniel A. Kane, Mohamed Touaibia, Erin E. Kershaw, Thomas Pulinilkunnil, Petra C. Kienesberger, Autotaxin Is Regulated by Glucose and Insulin in Adipocytes, Endocrinology, Volume 158, Issue 4, 1 April 2017, Pages 791-803" disclose that the PPARy agonist and ATX inhibitor, rosiglitazone for use in the treatment of fibrosis, cancer, NAFLD and diabetes. Document WO2010/040130A1 discloses rosiglitazone for use in treating lung diseases, particularly cystic fibrosis.

Document EP0306228 A1, discloses compounds for use in the treatment of type II diabetes.

Document "Sohda T, Mizuno K, Imamiya E, Sugiyama Y, Fujita T, Kawamatsu Y. Studies on antidiabetic agents. II. Synthesis of 5-[4-(1-methylcyclohexylmethoxy)-benzyl]thiazolidine-2,4-dione (ADD-3878) and its compounds. Chem Pharm Bull (Tokyo). 1982 Oct;30(10):3580-600" discloses on thiazolidinedione compounds as antidiabetic agents.

However, there is no reference in the prior art regarding the prevention or the treatment of
a) fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD,
via the simultaneous dual action of ATX inhibition and PPARγ agonism.

New pharmaceutical compounds were synthesized which incorporate in their structure both the functional group of thiazolidinediones with PPARγ agonism activity and the functional groups of strong ATX inhibitors PF8380, GLPG1690 and HA-155. Of the new synthesized compounds, only the pharmaceutical compounds having a chemical formula selected from the chemical formulas **(A-1), (A-2)** and **(A-3)** as described in the present invention, carrying a structurally similar group to the functional groups of PF8380, GLPG1690 showed strong autotaxin inhibition activity at the nanomolecular scale (IC₅₀= 0,19 µM and IC₅₀= 0,47 µM respectively), vs. IC₅₀= 20,67 µM of structurally similar group to HA-155.

It is a surprise that the pharmaceutical compounds having a chemical formula selected from the chemical formulas **(A-1), (A-2)** and **(A-3)** of the present invention, exhibit both PPARγ agonism and autotaxin inhibition properties.

It is a surprise that the new pharmaceutical compounds having a chemical formula selected from the chemical formulas **(A-1), (A-2)** and **(A-3)** of the present invention exhibit impressive results in each of the abovementioned treatments, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results in the anti-fibrotic treatment of interstitial lung diseases (ILD), primary or comorbid to autoimmune and/or inflammatory and/or metabolic diseases, through simultaneous PPARγ agonism and autotaxin inhibition.

It is a surprise that that the pharmaceutical compounds having a chemical formula selected from the chemical formulas **(A-1), (A-2)** and **(A-3)** of the present invention show impressive results in the treatment of bleomycin-induced pulmonary fibrosis and Idiopathic pulmonary fibrosis, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results against scleroderma, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results against rheumatoid arthritis, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results in the simultaneous treatment of pulmonary fibrosis and rheumatoid arthritis, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results in the simultaneous treatment of pulmonary fibrosis and scleroderma, through simultaneous PPARγ agonism and autotaxin inhibition.

It is a surprise that the new pharmaceutical compounds having a chemical formula selected from the chemical formulas **(A-1), (A-2)** and **(A-3)** of the present invention exhibit impressive results in the simultaneous treatment of pulmonary fibrosis and diabetes, through simultaneous PPARγ agonism and autotaxin inhibition.

It is a surprise that, in contrast to other thiazolidinedione compounds for use in the treatment of diabetes mellitus, the novel pharmaceutical compounds having a chemical formula selected from the chemical formulas **(A-1), (A-2)** and **(A-3)** of the present invention do not exhibit cardiotoxicity.

It is a surprise that the new pharmaceutical compounds having a chemical formula selected from the chemical formulas **(A-1), (A-2)** and **(A-3)** of the present invention do not exhibit hepatotoxicity.

The present invention is defined by the following definitions:
Definition 1. A pharmaceutical compound or pharmaceutically acceptable salt thereof, for use in the prevention or the treatment of
   fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD
   due to simultaneous Autotaxin (ATX) inhibition and PPARγ agonism, having the chemical formula selected from the chemical formulas (A-1), (A-2) and (A-3) 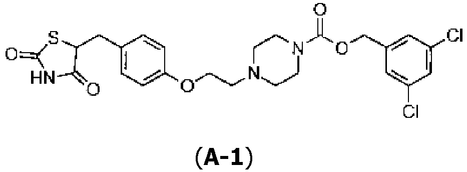 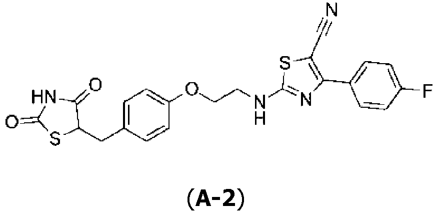 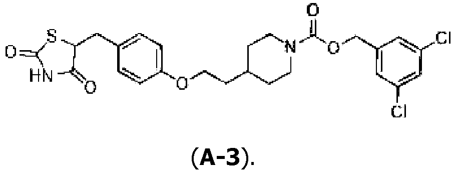
Definition 2. A pharmaceutical composition comprising a compound having the chemical formula selected from the chemical formulas (A-1), (A-2) and (A-3) 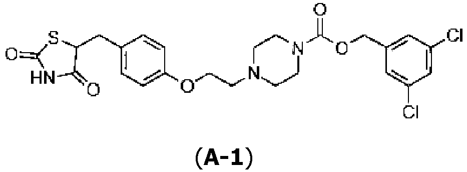 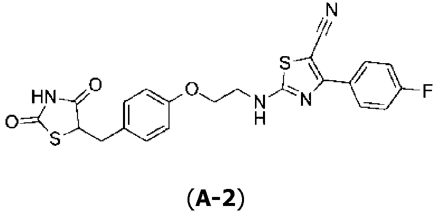 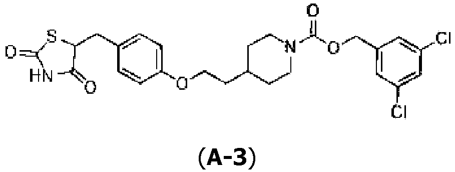 , furthermore, comprising one or more pharmaceutically acceptable excipients, for use in the prevention or the treatment of
   fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD.
Definition 3. The pharmaceutical composition for use according to definition 2, formulated with one or more excipientsthe method comprising inhalational or intraperitoneal or oral or intranasal or subcutaneous or intravenous or topical administration to a subject in need thereof, preferably inhalational administration to a subject in need thereof.

It was surprisingly found that the pharmaceutical compound **A-1** exhibits inhibitory activity against ATX with an IC₅₀ value of 0,19 µM when tested in well-established experimental *in vitro* protocols, outperforming existing non-toxic inhibitors.

It was surprisingly found that the pharmaceutical compound **A-1** exhibits impressive results in each of the abovementioned treatments.

It was surprisingly found that the pharmaceutical compound **A-1** has a clear indication in the anti-fibrotic treatment of interstitial lung diseases (ILD), primary or comorbid to autoimmune and/or inflammatory and/or metabolic diseases.

It was surprisingly found that the pharmaceutical compound **A-1** has impressive efficacy in the treatment of pulmonary fibrosis and comorbid interstitial lung disease and/or lung allograft fibrosis, due to simultaneous autotaxin inhibition and PPARγ agonism.

It was surprisingly found that the pharmaceutical compound **A-1** has impressive results in the treatment of pulmonary or cardiac fibrosis after type 2 diabetes mellitus.

It was surprisingly found that the pharmaceutical compound **A-1** has impressive results in the treatment of rheumatoid arthritis associated pulmonary fibrosis.

It was surprisingly found that the pharmaceutical compound **A-1** has impressive results in the treatment of combined skin and pulmonary fibrosis.

It was surprisingly found that the pharmaceutical compound **A-1** does not exhibit cardiotoxicity.

It was surprisingly found that the pharmaceutical compound **A-1** does not exhibit hepatotoxicity.

It was surprisingly found that compound **A-1** exhibits a very favorable pharmacokinetic profile.

It was surprisingly found that the pharmaceutical compound **A-2** exhibits inhibitory activity against ATX with an IC₅₀ value of 0,47 µM when tested in well-established experimental *in vitro* protocols, outperforming existing non-toxic inhibitors.

It was surprisingly found that the pharmaceutical compound **A-2** exhibits impressive results in each of the abovementioned treatments.

It was surprisingly found that the pharmaceutical compound **A-2** has a clear indication in the anti-fibrotic treatment of interstitial lung diseases (ILD), primary or comorbid to autoimmune and/or inflammatory and/or metabolic diseases.

It was surprisingly found that the pharmaceutical compound **A-2** has impressive efficacy in the treatment of pulmonary fibrosis and comorbid interstitial lung disease and/or lung allograft fibrosis, due to simultaneous autotaxin inhibition and PPARγ agonism.

It was surprisingly found that the pharmaceutical compound **A-2** has impressive results in the treatment of pulmonary or cardiac fibrosis after type 2 diabetes mellitus.

It was surprisingly found that the pharmaceutical compound **A-2** has impressive results in the treatment of rheumatoid arthritis associated pulmonary fibrosis.

It was surprisingly found that the pharmaceutical compound **A-2** has impressive results in the treatment of combined skin and pulmonary fibrosis.

It was surprisingly found that the pharmaceutical compound **A-2** does not exhibit cardiotoxicity.

It was surprisingly found that the pharmaceutical compound **A-2** does not exhibit hepatotoxicity.

It was surprisingly found that compound **A-2** exhibits a very favorable pharmacokinetic profile.

It was surprisingly found that the pharmaceutical compound **A-3** exhibits inhibitory activity against ATX with an IC₅₀ value of 0,03 µM when tested in well-established experimental *in vitro* protocols, outperforming existing non-toxic inhibitors.

It was surprisingly found that the pharmaceutical compound **A-3** exhibits impressive results in each of the abovementioned treatments.

It was surprisingly found that the pharmaceutical compound **A-3** has a clear indication in the anti-fibrotic treatment of interstitial lung diseases (ILD), primary or comorbid to autoimmune and/or inflammatory and/or metabolic diseases.

It was surprisingly found that the pharmaceutical compound **A-3** has impressive efficacy in the treatment of pulmonary fibrosis and comorbid interstitial lung disease and/or lung allograft fibrosis, due to simultaneous autotaxin inhibition and PPARγ agonism.

It was surprisingly found that the pharmaceutical compound **A-3** has impressive results in the treatment of pulmonary or cardiac fibrosis after type 2 diabetes mellitus. It was surprisingly found that the pharmaceutical compound **A-3** has impressive results in the treatment of rheumatoid arthritis associated pulmonary fibrosis.

It was surprisingly found that the pharmaceutical compound **A-3** has impressive results in the treatment of combined skin and pulmonary fibrosis.

It was surprisingly found that the pharmaceutical compound **A-3** does not exhibit cardiotoxicity.

It was surprisingly found that the pharmaceutical compound **A-3** does not exhibit hepatotoxicity.

It was surprisingly found that compound **A-3** exhibits a very favorable pharmacokinetic profile.

According to the present invention, the pharmaceutical compounds having a chemical formula selected from the chemical formulas **(A-1), (A-2)** and **(A-3)** can be mixed with suitable pharmaceutical acceptable excipients and formulated in various pharmaceutical forms and in an appropriate amount, in order to exhibit the intended therapeutic effect.

The present invention is described by the following representative but non-limiting examples:

### Example 1: Synthesis and activity evaluation of the pharmaceutical compound A-1.

For the synthesis of the pharmaceutical compound **A-1,** the synthetic route **Scheme 1** is followed.

The synthesis of the pharmaceutical compound **A-1** is described as following:

### -Synthesis of 4-(2-bromoethoxy)benzaldehyde (i):

4-hydroxybenzaldehyde (0.75 g, 6.14 mmol) is dissolved in dry CH₃CN (45 mL) and 1,2-dibromoethane (5.29 mL, 61.4 mmol) and K₂CO₃ (1.55 g, 11.2 mmol) are added. The mixture is stirred under reflux for 20h, cooled to rt, water (45 mL) is added and the mixture is extracted with Et₂O (2 x 30 mL). The combined organic phase is washed with brine (25 mL), dried (Na₂SO₄), filtered and concentrated in vacuum. The residue is recrystallized from Et₂O:hexane to give the product as a white solid. Yield = 0.92 g (65%). ¹H-NMR (CDCl₃, 400 MHz) *δ* 3.69 (td, J₁ = 1.7 Hz, J₂ = 6.2 Hz, 2H), 4.40 (td, J₁ = 1.7 Hz, J₂ = 6.2 Hz, 2H), 7.04 (dd, J₁ = 1.7 Hz, J₂ = 8.7 Hz, 2H), 7.87 (dd, J₁ = 1.9 Hz, J₂ = 8.7 Hz, 2H), 9.92 (s, 1H). MS [ESI+] m/z 229.9 [M + H]⁺.

### -Synthesis of 3,5-dichlorobenzyl piperazine-1-carboxylate hydrochloride (ii):

4N HCl in dioxane (16 mL, 63 mmol) was added to 1-(tert-butyl) 4-(3,5-dichlorobenzyl) piperazine-1,4-dicarboxylate **(iii,** 2.44 g, 6.27 mmol), whose synthesis is described below, at 0 °C and the reaction mixture is stirred at rt for 3h. The solvent is evaporated under reduced pressure and the white solid remaining is used in the next step without further purification. Yield = 2 g (quant.). ¹H-NMR (CDCl₃, 400 MHz) *δ* 3.09 (m, 4H), 3.65 (m, 4H), 5.10 (s, 2H), 7.47 (s, 2H), 7.57 (s, 1H), 9.49 (brs, 2H). MS [ESI+] m/z 326.1 [M + H]⁺.

### -Synthesis of 1-(tert-butyl) 4-(3,5-dichlorobenzyl) piperazine-1,4-dicarboxylate (iii):

To a solution of (3,5-dichlorophenyl)methanol (1.50 g, 8.47 mmol) in dry DMF (15 mL) is added CDI (1.92 g, 11.86 mmol) and the reaction is stirred at 45 °C for 2h. Then, 1-boc-piperazine (1.97 g, 10.59 mmol) is added and the reaction mixture is stirred at rt overnight. Water (30 mL) is added to the mixture and the precipitate is filtered, washed with water (2 x 10 mL) and hexane (10 mL) and dried. The crude product (white solid) is used immediately in the next step without further purification. Yield = 3.30 g (74%). MS [ESI+] m/z 390.1 [M + H]⁺.

### -Synthesis of 3,5-dichlorobenzyl 4-(2-(4-formylphenoxy)ethyl)piperazine-1-carboxylate (iv):

A mixture of compounds **i** (0.92 g, 4.02 mmol), **ii** (0.44 g, 4.42 mmol) and NaHCO₃ (1.35 g, 16.08 mmol) in dry DMF (20 mL) is stirred at 80 °C for 24 h and at 55 °C for 12 h. Then water (50 mL) is added and the mixture is extracted with ethyl acetate (3 x 25 mL). The combined organic phase is washed with water (25 mL) and brine (25 mL), dried (Na₂SO₄), filtered and concentrated in vacuum. The residue is purified by flash column chromatography eluted with hexane:EtOAc (7:3 to 100% ethyl acetate), affording an yellowish oil which solidifies upon standing in the fridge. Yield = 1.76 g (71%). ¹H-NMR (dmso-d₆, 400 MHz) *δ* 2.50-2.52 (m, 3H), 2.78 (t, J = 5.6 Hz, 2H), 2.97 (t, J = 5.6 Hz, 1H), 3.46 (m, 4H), 4.27 (t, J = 5.6 Hz, 2H), 5.11 (s, 2H), 7.12 (d, J = 8.4 Hz, 2H), 7.42 (s, 2H), 7.66 (d, J = 8.1 Hz, 2H), 7.70 (s, 1H), 9.90 (s, 1H). MS [ESI+] m/z 424.2 [M + H]⁺.

### -Synthesis of 3,5-dichlorobenzyl (E)-4-(2-(4-((2,4-dioxothiazolidin-5-ylidene)methyl)phenoxy)ethyl)piperazine-1-carboxylate (2):

In an oven-dried round-bottom flask, compound **iv** (1.68 g, 3.85 mmol) and 2,4-thiazolidinedione (0.54 g, 4.62 mmol) are dispersed in dry toluene (16 mL). Then, piperidine (0.20 mL, 1.92 mmol) is added followed by acetic acid (0.11 mL, 1.92 mmol) and the mixture is refluxed overnight. The reaction mixture is left to cool at rt where a brownish solid precipitates. The mixture is filtered, washed with toluene (20 mL) and hexane (20 mL) and dried at 50 °C overnight. Off-yellow powder. Yield = 2 g (quant.). ¹H-NMR (dmso-d₆, 400 MHz) *δ* 2.50-2.52 (m, 3H), 2.76 (t, J = 5.6 Hz, 2H), 2.99 (t, J = 5.6 Hz, 1H), 3.42 (m, 4H), 4.17 (t, J = 5.6 Hz, 2H), 5.08 (s, 2H), 7.10 (d, J = 8.4 Hz, 2H), 7.42 (s, 2H), 7.53 (s, 1H), 7.56 (d, J = 8.1 Hz, 2H), 7.70 (s, 1H). MS [ESI+] m/z 537.3 [M + H]⁺.

### -Synthesis of 3,5-dichlorobenzyl4-(2-(4-((2,4-dioxothiazolidin-5-yl)methyl)phenoxy)ethyl)piperazine-1-carboxylate (A-1):

The thiazolidinone compound **2** (0.45 g, 0.84 mmol) is mixed with water (25 mL), to which 5 drops of 0.5M aqueous solution of NaOH is added until pH 11. Then, a mixture of THF:DMF 2:1 (20 mL) is added followed by CoCl₂ 6H₂O (0.128 g, 0.537 mmol), dimethylglyoxime (0.129 g, 1.107 mmol) and sodium borohydride (0.374 g, 9.88 mmol). The reaction mixture is stirred at room for 24h, where a partial conversion of the starting material to the desired product was observed by TLC and MS. Then, more CoCl₂ 6H₂O (0.128 g, 0.537 mmol), dimethylglyoxime (0.129 g, 1.107 mmol) and sodium borohydride (0.374 g, 9.88 mmol) were added and the mixture was stirred overnight. The pH of the reaction was adjusted to 3 with 6N HCl and then to 10 by adding 1N NaOH. It was extracted with ethyl acetate (2 x 50 mL), the combined organic phase was washed with water (30 mL) and brine (30 mL), dried (Na₂SO₄), filtered and concentrated in vacuum. The residue was purified by flash column chromatography eluted with ethyl acetate, providing the desired product **(A-1)** as a slight yellow semisolid. Yield = 0.226 g (50%). ¹H-NMR (dmso-d₆, 400 MHz) *δ* 2.45-2.49 (m, 5H), 2.72 (q, J = 5.0 Hz, 2H), 3.00-3.09 (m, 1H), 3.37-3.48 (m, 4H), 4.06 (q, J = 5.0 Hz, 2H), 4.86 (dt, J₁ = 3.8 Hz, J₂ = 8.5 Hz, 1H), 5.09 (s, 2H), 6.89 (dd, J₁ = 3.2 Hz, J₂ = 8.5 Hz), 7.15 (dd, J₁ = 3.1 Hz, J₂ = 8.4 Hz), 7.43 (s, 2H), 7.57 (s, 1H), 11.96 (brs, 1H). MS [ESI+] m/z 539.1 [M + H]⁺.

### ATX inhibitory activity assessment

The inhibition of ATX enzyme activity of the pharmaceutical compound **A-1** was measured *in vitro* by applying the Amplex Red method. Briefly, 2 µL of concentration from 0,001 to 5 µmol/L of compound (A4) in DMSO was incubated with 50 µL ATX 8 nM (up to a final concentration of 2 nM and 1 nM for human ATX) and 48 µL of a buffer solution (50 mM Tris-Cl of pH 8.0 and 5 mM CaCl₂) for 15 minutes at 37 °C. Subsequently, 50 µL of a buffer solution containing 200 µM LPC 16:0 (final concentration 50 µM) was added to the reaction mixture followed by incubation for another 30 minutes at 37 °C. Finally, 50 µL of working solution containing 200 µM Amplex Red reagent and choline oxidase (0.2 U/mL) κα 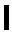 HRP (2 U/mL), in 50 mM Tris-HCl at pH 8.0 and 5 mM CaCl₂, was added to begin the reaction. The reaction was monitored at 37 °C, in a fluorescence plate reader (Tecan Infinite 200) using excitation at 530 nm and reading at 590 nm, every five minutes for 30 minutes at 37°C. The IC₅₀ value was calculated from two independent experiments, using the sigmoid dose-response curve (PrismH software) from the equation f=y0+a/(1+exp(-(x-x0)/b) given from SigmaPlot 11.0.

To determine the mode of inhibition of ATX by each inhibitor, a modified Amplex assay was employed. In brief, various concentrations of inhibitors (1, 2.5, 5 and 7.5 µM) were tested against various substrate concentrations (LPC 25, 50, 100 µM). Mouse ATX from Sino Biological was used in all mode of inhibition assays. Thereafter, the velocity (V) of the reaction was calculated and the reciprocal of velocity (1/V) was plotted against the reciprocal of substrate concentration (1/S) in Lineweaver-Burk graphs using GraphPad Software (GraphPad Software, San Diego, CA, USA).

In **Scheme 2** it is shown that drug compound **A-1** has a lower IC₅₀ (0.19 µM) compared to TGL (0.61 µM). Also, as shown in **Scheme 3,** compound **A-1** exhibits non-competitive inhibition against ATX. In addition, **A-1** showed no inhibition against the enzymes choline oxidase and HRP peroxidase as shown in **Scheme 4,** thus it is a specific inhibitor of ATX.

### In vitro ADMET (absorption, distribution, metabolism, excretion, and toxicity) assays

The physicochemical properties evaluated were: *in vitro* membrane permeability, *in vitro* metabolism and cardiotoxicity.

The ***in vitro* absorption assay** was performed in the kidney MDCKII cell line and involves the permeability from A to B, when A and B are separated by an MDCKII cell membrane. Ideally, a compound should have a high A to B permeability, but a lower B to A permeability in order to be well-absorbed. A-B permeability of compound **A-1** was calculated to be 3.7 x10⁻⁶ cm/s, whereas B-A permeability was found to be 0.8 x10⁻⁶ cm/s. The A-B permeability is much higher than the B-A permeability, thus both compounds are absorbed well *in vitro.*

The ***in vitro metabolism* assay** was performed in liver microsomes. It was calculated that compound **A-1** exhibits a half-life (t_{1/2}) of 21 minutes. This half-live is higher than the ones of known drugs such as imipramine and others, therefore, it seems that **A-1** is not quickly cleared from the circulation and, thus, remain available in the circulation for a sufficient period of time. This conclusion is further verified by the low Clint values, a parameter indicative of intrinsic clearance (328,6 µL/min/mg for compound **A-1,** lower than the Clint of many other drugs).

The **assay of cardiac toxicity** is performed on CHO cells that express the human ether-a-go-go related gene (hERG) that encodes the inward rectifying voltage gated potassium channel in the heart, which is involved in cardiac repolarisation. Inhibition of the hERG may cause potentially fatal ventricular tachyarrhythmia. The IC₅₀ of the pharmaceutical compound **A-1** relative to hERG inhibition was estimated to be 12 µM **(Scheme 5).** This value is quite high (higher than their IC₅₀ₛ against ATX) and, therefore the pharmaceutical compound **A-1** is not cardiotoxic.

### In vivo evaluation of the pharmaceutical compound A-1 on pulmonary fibrosis in mice

10-week-old male wild-type C57BI6/J animals were administered a saline solution of bleomycin (BLM) directly to the lungs via the orotracheal route. The dose of bleomycin was 0,8U/kg. The pharmaceutical compound **A-1** was administrated in doses of 30 mg/kg and each animal received 2 doses daily starting one day before the infusion of BLM. All animals were monitored daily until the 14th day after the BLM injection when they were killed. The group that received the pharmaceutical compound **A-1** did not suffer any unwanted side effects compared to the untreated group during the model. Serum, broncheoalveolar fluid and lung tissue samples were taken at the end point of the experiment and were used to assess the severity of fibrosis for each animal.

Blood serum was collected for each animal and levels of ALT and AST transaminases, which are markers of liver damage, were assessed by using an automated biochemical analyzer. As shown in **Scheme 6,** the levels of ALT and AST appear to be normal in all five groups (within the normal ranges that are 28-132 U/L and 59-247 U/L for ALT and AST, respectively). These results verify that the pharmaceutical compound **A-1** does not cause any adverse toxicity to the liver of the animals.

Next, in the collected broncheoalveolar fluid, the number of cells as well as the total protein concentration were assessed as indicators of inflammation and endothelial penetration, respectively. As shown in **Scheme 7,** the group that received the new pharmaceutical compound **A-1** (B+A-1) shows a reduced number of cells in the bronchoalveolar fluid and lower levels in the total protein concentration compared to the group that received only the vehicle of the compounds (B+V).

Additionally, in sections of lung tissue isolated from the animals, fibrosis was assessed after staining with hematoxylin and eosin. **Scheme 8** shows representative images of each group. The group B+V that received no ATX inhibitor treatment appears to have distinct fibrotic areas in contrast to the control group that received the new pharmaceutical compound **A-1,** which undoubtedly presents less fibrotic areas.

The mRNA levels of fibrosis-related genes col1α1 (collagen 1α1) in whole lung RNA samples were also estimated. As shown in **Scheme 9,** the new pharmaceutical compound **A-1** reduces the mRNA levels (expression) of col1α1 statistically significantly, and even restores them to the same levels as the control group that received saline (S + V).

The pharmaceutical compound **A-1** of the above example has an excellent effect in the simultaneous treatment of type 2 diabetes mellitus on the one hand and restrictive lung disease and/or pulmonary fibrosis and/or pulmonary sarcoidosis and/or lung allograft fibrosis on the other, as well as in reducing cardiotoxicity and hepatotoxicity, and fibrosis phenomena after type 2 diabetes mellitus.

To explore the therapeutic potential of ATX inhibition in pulmonary fibrosis, BLM was administered to 8-10-week-old C57BI6/J mice. Subsequently, the pharmaceutical compound **A-1** was delivered for 7 days twice daily via inhalation (1ml of 6,5 mg/ml for 10 mins/6 mice, corresponding to 15 mg/Kg per mouse) to conscious, gently restrained mice, in a therapeutic mode (7d post BLM). Inflammatory cells in the bronchoalveolar lavage fluid (BALF), as measured by hematocytometer, were found significantly reduced in WT mice post BLM, upon ATX inhibition **(Scheme 10).** Furthermore, the pharmaceutical compound **A-1** administration led to a noteworthy decrease in vascular leak and pulmonary edema, as indicated by the total protein concentration **(Scheme 10).** Histological analysis revealed that ATX inhibition prevented BLM-induced architectural distortion, as indicated with H&E staining **(Scheme 11).** The relative protection from the BLM-induced tissue architecture distortion upon ATX inhibition, was also reflected in lung respiratory functions, as measured with FlexiVent **(Scheme 12).** Overall, these findings underscore the substantial impact of ATX expression in the context of BLM-induced pulmonary fibrosis, suggesting its potential relevance to IPF.

### Example 2: Synthesis and activity evaluation of the pharmaceutical compound A-2.

For the synthesis of the pharmaceutical compound **A-2,** the synthetic route **Scheme 13** is followed.

The synthesis of compound **A-2** is described as following:
- **Synthesis of tert-butyl (2-(4-formylphenoxy)ethyl)carbamate (v):** To a solution of 2-(boc-amino)ethanol (1 g, 6.20 mmol), 4-hydroxybenzaldehyde (0.91 g, 7.44 mmol) and triphenyl phosphine (2.44 g, 9.30 mmol) in dry THF (25 mL) is added di-isopropyl azodicarboxylate (1.83 mL, 9.30 mmol) dropwise at 0 °C. The mixture is then allowed to stir at rt for 2.5 h. The solvent is removed under vacuum, the residue is dissolved in ethyl acetate (50 mL), washed with 1N NaOH (10 mL), water (10 mL) and brine (10 mL), dried (Na₂SO₄), filtered and concentrated in vacuum. The residue is purified by flash column chromatography eluted with hexane:ethyl acetate (4:1), furnishing the desired product as a white solid. Yield = 1.64 g (quant.). ¹H-NMR (CDCl₃, 400 MHz) *δ* 1.46 (s, 9H), 3.58 (d, J = 4.5 Hz, 2H), 4.12 (t, J = 5.0 Hz, 2H), 5.10 (brs, 1H), 7.01 (d, J = 8.5 Hz, 2H), 7.84 (d, J = 9.2 Hz, 2H), 9.89 (s, 1H). MS [ESI+] m/z 266.1 [M + H]⁺.
- **Synthesis of tert-butyl (E)-(2-(4-((2,4-dioxothiazolidin-5-ylidene)methyl)phenoxy)ethyl)carbamate (vi):** To a solution of compound **v** (1.50 g, 5.65 mmol) in dry toluene (15 mL), 2,4-thiazolidinone (0.80 g, 6.79 mmol) is added, followed by piperidine (0.28 mL, 2.83 mmol) and acetic acid (0.162 mL, 0.83 mmol). The mixture is stirred at reflux for 8 h and then is allowed at rt overnight. The resulting solid precipitated is filtered, washed with toluene (3 mL) and hexane (5 mL), and dried at 50 °C overnight to give the desired product as a beige/brownish amorphous solid. Yield =1.63 g (80%). ¹H-NMR (DMSO-d₆, 400 MHz) *δ* 1.38 (s, 9H), 3.31 (m, 2H), 4.06 (m, 2H), 7.01 (s, 1H), 7.10 (d, J = 7.4 Hz, 2H), 7.55 (d, J = 8.7 Hz, 2H), 7.75 (s, 1H), 12.49 (brs, 1H). MS [ESI+] m/z 365.2 [M + H]⁺.
- **Synthesis of tert-butyl (2-(4-((2,4-dioxothiazolidin-5-yl)methyl)phenoxy)ethyl)carbamate (vii):** The thiazolidinone compound **vi** (0.80 g, 2.20 mmol) and magnesium turnings (1.07 g, 43.91 mmol) are added in a flask and air is removed in vacuum. Then Argon is placed in the flask and anhydrous methanol (27 mL) is added. The mixture is stirred at rt and under Ar for 4h. The reaction mixture is acidified with 6N HCl to pH 5-6 and extracted with dichloromethane (2 x 25 mL) and the combined organic phase is washed with water (15 mL) and brine (15 mL), dried (Na₂SO₄), filtered and concentrated in vacuum. The residue was purified by flash column chromatography eluted with hexane:ethyl acetate (3:2), affording the desired compound as a yellow oil. Yield = 0.37 g (46%). ¹H-NMR (CDCl₃, 400 MHz) *δ* 1.48 (s, 9H), 3.12 (dd, J₁ = 3.5 Hz, J₂ = 14 Hz, 1H), 3.47 (dd, J₁ = 3.5 Hz, J₂ = 14.0 Hz, 1H), 3.55 (brd, J = 4 Hz, 2H), 4.03 (t, J = 5 Hz, 2H), 4.51 (dd, J₁ = 3.5 Hz, J₂ = 9.5 Hz, 1H), 5.06 (brs, 1H), 6.87 (d, J = 8.0 Hz, 2H), 7.17 (d, J = 8.0 Hz, 2H), 8.96 (brs, 1H). MS [ESI+] m/z 367.1 [M + H]⁺.
- **Synthesis of compounds viii and ix:** A solution of 4N HCl in dioxane (1.90 mL, 7.61 mmol) is added at once to compounds **vi** and **vii** (0.22 g, 0.61 mmol) and the mixture is stirred at rt for 4h. The solvent is distilled in vacuum, the residue is washed with diethyl ether (15 mL) and dried, providing the desired product as a white solid.
   **(E)-5-(4-(2-aminoethoxy)benzylidene)thiazolidine-2,4-dione hydrochloride (viii):** Yield = 0.111 g (quant.). ¹H-NMR (CH₃OD, 400 MHz) *δ*. 3.45 (dd, J₁ = 4.2 Hz, J₂ = 14.1 Hz, 1H), 4.55 (brs, 2H), 6.98 (d, J = 8.0 Hz, 2H), 7.23 (d, J = 8.0 Hz, 2H), 7.91 (s, 1H). MS [ESI+] m/z 302.0 [M + H]⁺.
   **5-(4-(2-aminoethoxy)benzyl)thiazolidine-2,4-dione hydrochloride (ix):** Yield = 0.115 g (quant.). ¹H-NMR (CH₃OD, 400 MHz) *δ* 3.16 (dd, J₁ = 9.0 Hz, J₂ = 14.0 Hz, 1H), 3.38 (brs, 2H), 3.41 (dd, J₁ = 4.0 Hz, J₂ = 14.0 Hz, 1H), 4.23 (brs, 2H), 4.73 (dd, J₁ = 4.0 Hz, J₂ = 9.5 Hz, 1H), 6.98 (d, J = 8.0 Hz, 2H), 7.23 (d, J = 8.0 Hz, 2H). MS [ESI+] m/z 304.1 [M + H]⁺.
- **Synthesis of 2-chloro-4-(4-fluorophenyl)thiazole-5-carbonitrile (x):** To a solution of anhydrous CuCl₂ (0.47 g, 3.47 mmol) in dry CH₃CN (6.5 mL) is added dropwise tert-butoxy nitrite (0.45 g, 4.34 mmol) and the mixture is stirred at rt for 45 min. Then, compound **xi** (0.63 g, 2.89 mmol) is added in portions and stirring is continued for further 2h. The reaction mixture is carefully quenched with 1N HCl (10 mL) and stirred for 15 min. The organic phase is separated, the aqueous phase is extracted with ethyl acetate (20 mL) and the combined organic phase is washed with brine (10 mL), dried (Na₂SO₄), filtered and concentrated in vacuum. The crude product is filtered on a silica plug eluted with dichloromethane. Solvents are distilled in vacuum and the residue is triturated with hexane, filtered and dried. The product is isolated as a bright orange thick solid. Yield = 0.49 g (71%). ¹H-NMR (CDCl₃, 400 MHz) *δ* 7.19-7.25 (m, 2H), 8.12-8.17 (m, 2H). MS [ESI+] m/z 240.0 [M + H]⁺.
- **Synthesis of 2-amino-4-(4-fluorophenyl)thiazole-5-carbonitrile (xi):** To a solution of 4-fluorobenzoylacetonitrile (0.47 g, 2.89 mmol) in dry ethanol (6 mL) dry pyridine (0.24 mL, 2.89 mmol) is added and the mixture is stirred at 70 °C for 20 min and then cooled to rt. A previously stirred suspension of thiourea (0.44 g, 5.79 mmol) and iodine (0.73 g, 2.89 mmol) in dry ethanol (4 mL) is slowly added and the mixture is stirred at rt for 2h. Cold water (40 mL) is added and the resulting precipitate is filtered, washed with water (10 mL) and hexane (15 mL) and dried in vacuum to afford the desired product as a yellow solid. Yield = 0.63 g (quant). ¹H-NMR (dmso-d₆, 400 MHz) *δ* 7.37 (t, J = 8.9 Hz, 2H), 7.93-8.01 (m, 2H), 8.25 (s, 2H). MS [ESI+] m/z 220.0 [M + H]⁺.
- **Synthesis of compounds 3 and 4:** Compounds **viii or ix** (0.25 g, 0.83 mmol) and **x** (0.18 g, 0.76 mmol) are dissolved in dry DMSO (7 mL), DIPEA (0.33 mL, 1.89 mmol) is added and the mixture is stirred at 100 °C for 8 h and at rt overnight. Water (15 mL) is added and the mixture is extracted with ethyl acetate (2 x 30 mL). The combined organic phase is washed with water (2 x 20 mL) and brine (20 mL), dried (Na₂SO₄), filtered and concentrated in vacuum. The residue is purified by flash column chromatography eluted with hexane:ethyl acetate (7:3 to 1:1) furnishing the final products.
   **2-((2-(4-((2,4-dioxothiazolidin-5-yl)methyl)phenoxy)ethyl)amino)-4-(4-fluorophenyl)thiazole-5-carbonitrile (4).** Off-yellow amorphous solid. Yield = 0.20 g (56%). ¹H-NMR (dmso-d₆, 400 MHz) *δ*. MS [ESI+] m/z 469.1 [M + H]⁺
   **(E)-2-((2-(4-((2,4-dioxothiazolidin-5-ylidene)methyl)phenoxy)ethyl)amino)-4-(4-fluorophenyl)thiazole-5-carbonitrile (3)(compound A-2).** Light brown solid. Yield = 0.34 g (95%). ¹H-NMR (dmso-d₆, 400 MHz) *δ* 2.51-2.52 (m, 6H), 3.29-33.8 (m, 4H), 3.79-3.82 (m, 2H), 4.28 (s, 2H), 7.14 (d, J = 8.5 Hz, 2H), 7.37 (t, J = 8.7 Hz, 2H), 7.55 (d, J = 8.4 Hz, 2H), 7.71 (s, 1H), 8.00 (dd, J₁ = 5.5 Hz, J₂ = 8.6 Hz, 2H), 9.02 (brs, 1H). MS [ESI+] m/z 467.1 [M + H]⁺.

### ATX inhibitory activity assessment

The inhibition of the enzymatic activity of ATX by the pharmaceutical compound **A-2** was performed as described above for the **Example 1** of the present invention.

In **Scheme 2** it is shown that compound **A-2** has a lower IC₅₀ (0,47 µM) compared to TGL (0,61 µM). Also, as shown in **Scheme 3,** the pharmaceutical compound **A-2** exhibits non-competitive inhibition against ATX. Furthermore, the pharmaceutical compound **A-2** showed no inhibition against the enzymes choline oxidase and HRP peroxidase as shown in **Scheme 4,** thus it is a specific inhibitor of ATX.

### In vitro ADMET (absorption, distribution, metabolism, excretion, and toxicity) assays

The physicochemical properties of the pharmaceutical compound **A-2** were evaluated as described in the **Example 1** of the present invention.

According to the ***in vitro* absorption test,** the A-B permeability of the pharmaceutical compound **A-2** was calculated to be 7,8 x10⁻⁶ cm/s, while the B-A permeability was found to be 3,2 x 10⁻⁶ cm/s, therefore the pharmaceutical compound **A-2** is absorbed well *in vitro.*

According to the ***in vitro metabolism* assay,** the pharmaceutical compound **A-2** was estimated to have a half-life (t_{1/2}) > 60 min, therefore, the pharmaceutical compound **A-2** is not rapidly cleared from the circulation and therefore remains available in the circulation for sufficient time. This conclusion is further verified by the low Clint value (< 115,5 µL/min/mg), which is lower than the Clint of many other drugs.

According to the **cardiac toxicity assay,** the IC₅₀ of the pharmaceutical compound **A-2** with respect to hERG inhibition was estimated to be >100 µM **(Scheme 5).** This value is very high (higher than the IC₅₀ against ATX) and therefore the pharmaceutical compound **A-2** is not cardiotoxic.

### In vivo evaluation of the pharmaceutical compound A-2 on pulmonary fibrosis in mice

The *in vivo* evaluation of pulmonary fibrosis in mice after administration of the pharmaceutical compound **A-2** was performed as described in the Example 1 of the present invention. The group that received the pharmaceutical compound **A-2** suffered no adverse side effects compared to the untreated group during the course of the model. As shown in **Scheme 6,** ALT and AST levels are normal (within the normal range of 28-132 U/L and 59-247 U/L for ALT and AST, respectively), thus the pharmaceutical compound **A-2** does not cause adverse animal liver toxicity. In addition, the group treated with the pharmaceutical compound **A-2** shows reduced cell numbers and total protein levels compared to the untreated group (B+V) **(Scheme 7).** Animals treated with A-2 (B+A-2) show milder fibrotic areas than the untreated group (B+V) **(Scheme 8).** Finally, the pharmaceutical compound A-2 significantly reduces the mRNA levels (expression) of col1α1, to the same levels as the control group that received saline (S + V), as shown in **Scheme 9.**

The pharmaceutical compound **A-2** of the above Example 2 has an excellent effect in the simultaneous treatment of type 2 diabetes mellitus on the one hand and on the other hand in restrictive lung disease and/or pulmonary fibrosis and/or pulmonary sarcoidosis and/or lung allograft fibrosis, as well as in reducing cardiotoxicity and hepatotoxicity, and fibrosis phenomena after type 2 diabetes mellitus.

**Example 3:** Synthesis and activity evaluation of the pharmaceutical compound **A-3.** For the synthesis of compound **A-3,** the synthetic route **Scheme 14** is followed.

The synthesis of the pharmaceutical compound **A-3** is described as following:
- **Synthesis of tert-butyl 4-(2-bromoethyl)piperidine-1-carboxylate (i):** In an oven-dried microwave vial employed with magnetic stirrer, N-Boc-4-piperidinethanol (0.521 g, 2.274 mmol) is dissolved in dichloromethane (10 mL). Then triphenylphosphine (0.835 g, 3.184 mmol) and carbon tetrabromide (1.207 g, 3.640 mmol) were added in portion and the reaction mixture was stirred at room temperature for 72h. Upon completion, the solvent was evaporated and the residue was purified by silica gel flash column chromatography eluted with hexane:ethyl acetate (100% hexane to 5% ethyl acetate in hexane). Colorless oil. Yield: = 0.530 g (79%).
- **Synthesis of tert-butyl 4-(2-(4-formylphenoxy)ethyl)piperidine-1-carboxylate (ii):** In a round-bottom flask with magnetic stirrer, tert-butyl 4-(2-bromoethyl)piperidine-1-carboxylate **(i)** (0.530 g, 1.814 mmol), 4-hydroxybenzaldehyde (0.277 g, 2.268 mmol), caesium carbonate (1.478 g, 4.535 mmol) and dry DMF (3 mL) were consecutively added and the reaction mixture was stirred at 70 °C for 6h and at room temperature overnight. Water (10 mL) was added and the mixture was extracted with ethyl acetate (3 x 20 mL). The combined extracts were washed with water (2 x 15 mL), saturated aqueous solution of sodium carbonate (2 x 10 mL) and brine (15 mL), dried (Na₂SO₄), fileterd and concentrated in vacuum. The product was purified by silica gel flash column chromatography eluted with hexane:ethyl acetate (EtOAc 0-20%). Off-white/yellowish solid. Yield = 0.535 g (88%).¹H-NMR (CDCl₃, 400 MHz) *δ* 1.17-1.28 (m, 2H), 1.48 (s, 9H), 1.76-1.82 (m, 5H), 2.66-2.76 (m, 2H), 4.12 (t, *J* = 6.2 Hz, 4H), 7.01 (d, *J* = 8.7 Hz, 2H), 7.86 (d, *J* = 8.8 Hz, 2H), 9.91 (s, 1H).
- **Synthesis of 4-(2-(piperidin-4-yl)ethoxy)benzaldehyde (iii):** tert-butyl 4-(2-(4-formylphenoxy)ethyl)piperidine-1-carboxylate **(ii)** (0.531 g, 1.593 mmol) was dissolved in dey dichloromethane (4 mL) in a round-bottom flask and trifluoroacetic acid (3.50 mL, 5.447 g, 47.776 mmol) was subsequently added. The reaction mixture was stirred at room temperature for 2h, the solvent was evaporated in vacuum, saturated sodium bicarbonate aqueous solution (5 mL) was added and the product was extracted with ethyl acetate (3 x 15 mL). The combined organic phase was washed with brine (15 mL), dried (Na₂SO₄), filtered and concentrated in vacuum. Yellowish semisolid. Yield = 0.300 g (81%).¹H-NMR (dmso-d₆, 400 MHz) *δ* 1.34-1.44 (m, 2H), 1.76-1.85 (m, 5H), 2.74 (t, *J* = 13.7 Hz, 2H), 3.23 (d, *J* = 12.3 Hz, 2H), 4.11 (t, *J* = 6.0 Hz, 2H), 5.20 (brs, 1H), 7.00 (d, *J* = 8.5 Hz, 2H), 7.85 (d, *J* = 8.7 Hz, 2H), 9.90 (s, 1H).
- **Synthesis of 3,5-dichlorobenzyl 4-(2-(4-formylphenoxy)ethyl)piperidine-1-carboxylate (iv):** To a solution of 3,5-dichlorobenzyl alcohol (0.284 g, 1.607 mmol) in dry DMF (2.3 mL), carbonyl di-imidazole (CDI, 0.365 g, 2.251 mmol) is added and the mixture is stirred at 45 °C for 3h. Then, 4-(2-(piperidin-4-yl)ethoxy)benzaldehyde **(iii)** (0.300 g, 1.286 mmol) is dissolved in dry DMF (2 mL) and added dropwise to the reaction mixture which is then stirred at 45 °C for 3h and at room temperature overnight. Water (15 mL) is added and the mixture is extracted with diethyl ether (3 x 10 mL). The combined organic phase is washed with water (12 mL) and brine (12 mL), dried (Na₂SO₄), filtered and concentrated in vacuum. The product was purified by silica gel flash column chromatography eluted with hexane-ethyl acetate (85:15 to 70:30). Off-white semisolid. Yield = 0.533 g (95%). ¹H-NMR (CDCl₃, 400 MHz) *δ* 1.23-1.30 (m, 2H), 1.79-1.82 (m, 5H), 2.77-2.95 (brm, 2H), 4.10-4.24 (m, 4H), 5.09 (s, 2H), 7.01 (d, *J* = 8.4 Hz, 2H), 7.25 (s, 2H), 7.32 (s, 1H), 7.86 (d, *J* = 8.5 Hz, 2H), 9.91 (s, 1H).
- **Synthesis of 3,5-dichlorobenzyl (*E*)-4-(2-(4-((2,4-dioxothiazolidin-5-ylidene)methyl)phenoxy)ethyl)piperidine-1-carboxylate (v):** In an oven-dried microwave vial employed with a magnetic stirrer, 3,5-dichlorobenzyl 4-(2-(4-formylphenoxy)ethyl)piperidine-1-carboxylate **(iv)** (0.521 g, 1.194 mmol) and 2,4-thiazolidinedione (0.168 g, 1.433 mmol) were placed followed by dry toluene (5 mL), piperidine (59.2 µL, 0.051 g, 0.597 mmol) and acetic acid (34.2 µL, 0.036 g, 0.597 mmol). The mixture was stirred under reflux (111 °C) overnight. The mixture was cooled to room temperature where a yellow solid precipitated. The solid was filtered, washed with toluene and hexane and dried at 50 °C overnight. Yellow powder. Yield = 0.500 g (78%).¹H-NMR (dmso-d₆, 400 MHz) *δ* 1.09-1.12 (m, 2H), 1.66-1.71 (m, 5H), 2.76-2.91 (brm, 2H), 4.00 (d, *J* = 13.2 Hz, 2H), 4.10 (t, *J* = 5.8 Hz, 2H), 5.07 (s, 2H), 7.10 (d, *J* = 8.8 Hz, 2H), 7.41 (s, 2H), 7.54-7.56 (m, 3H), 7.75 (s, 1H), 12.51 (brs, 1H).
- **Synthesis of 3,5-dichlorobenzyl 4-(2-(4-((2,4-dioxothiazolidin-5-yl)methyl)phenoxy)ethyl)piperidine-1-carboxylate (3)(A-3):**
   **Catalyst:** 9 mg (0.038 mmol) of of CoCl₂ 6H₂O and 49 mg (0.413 mmol) of dimethylglyoxime are dissolved in 0.55 mL DMF under stirring, yielding a clear blue-green solution.
   **Reducing agent:** 0,177 g (4.670 mmol) of NaBH₄ are dissolved in 1.50 mL H₂O+ 0.5 mL of 0.1 M solution of NaOH under cooling in ice (at 0 °C). It is maintained in ice until it is consumed.
   **Reaction:** 23.4 mg of NaOH and subsequently 0.250 g (0.467 mmol) of 3,5-dichlorobenzyl 4-(2-(4-((2,4-dioxothiazolidin-5-yl)methyl)phenoxy)ethyl)piperidine-1-carboxylate **(3)** are dissolved in 5 mL of H₂O and the obtained solution is stirred and heated at 55 °C until a solution is formed. The catalyst is added (0.15 mL of CoCl₂-DMG in DMF) during 1 min into the solution, followed by the reducing agent (0.50 mL of NaBH4 in H2O) during 2 min, and the mixture is stirred at 55 °C for 1 h. The same procedure is repeated 3 more times (each time, addition of 1/4 of the catalyst followed by 1/4 of the reducing agent, followed by 1 h stirring at 55 °C, i.e. 1 addition every 1 hour). Then the mixture is stirred at 45 °C overnight. The following day 10 mL of 6 N HCl were added. After extraction, the aqueous phase is further extracted with EtOAc (2x 20 mL). The combined organic phase is washed with water (20 mL) and brine (20 mL), dried, filtered and concentrated in vacuum. The product was purified via silica gel flash column chromatography eluted with hexane:EtOAc 3:2. White crystalline solid. Yield = 0.110 g (44%). ¹H-NMR (dmso-d₆, 400 MHz) *δ* 1.04-1.16 (m, 2H), 1.66-1.74 (m, 5H), 2.76-2.85 (brm, 2H), 3.06 (dd, *J₁ =* 14.1 Hz, *J₂ =* 9.0 Hz, 1H), 3.97-4.00 (m, 4H), 4.87 (dd, *J₁* = 9.0 Hz, *J₂ =* 4.3 Hz, 1H), 5.06 (s, 2H), 6.87 (d, *J* = 8.2 Hz, 2H), 7.14 (d, *J* = 8.3 Hz, 2H), 7.41 (s, 2H), 7.56 (s, 1H), 12.01 (brs, 1H). ¹³C-NMR (dmso-d₆, 100 MHz) *δ* 32.0, 32.6 (2C), 35.6, 36.7, 44.2 (2C), 53.5, 65.0, 65.5, 114.8 (2C), 126.6 (2C), 127.9, 128.9, 130.8 (2C), 134.5 (2C), 141.9, 154.5, 158.1, 172.2, 176.2. MS [ESI+] m/z 538.2 [M + H]⁺.

### ATX inhibitory activity assessment

The inhibition of the enzymatic activity of ATX by the pharmaceutical compound **A-3** was performed as described above for the **Example 1** of the present invention.

In **Scheme 2** it is shown that compound **A-3** has a lower IC₅₀ (0,03 µM) compared to TGL (0,61 µM). Also, as shown in **Scheme 3,** the pharmaceutical compound **A-3** exhibits non-competitive inhibition against ATX. Furthermore, the pharmaceutical compound **A-3** showed no inhibition against the enzymes choline oxidase and HRP peroxidase as shown in **Scheme 4,** thus it is a specific inhibitor of ATX. Of note, according to the cardiac toxicity assay, the pharmaceutical compound **A-3** did not show any significant inhibitory activity against hERG (11% inhibition at 25 µM), and therefore the pharmaceutical compound **A-3** is not cardiotoxic. It is the less cardiotoxic than any other ATX inhibitor, referred in the prior art.

With reference to the following attached Schemes 1 to 14 the above examples are described in more detail and the above-indicated results are better understood:
**Scheme 1.** Synthetic route of the pharmaceutical compound **A-1.**
**Scheme 2.** Inhibitory graphs of the pharmaceutical compounds **A-1, A-2, A-3** and troglitazone against ATX.
**Scheme 3.** Mode of inhibition for the pharmaceutical compounds **A-1, A-2, A-3** against ATX.
**Scheme 4.** Kinetic graphs for the 2^{nd} and 3^{rd} reactions of the Amplex Red assay in the presence of the pharmaceutical compounds **A-1, A-2, A-3.** Neither compound showed any detectable inhibition of the 2^{nd} (choline oxidase) or 3^{rd} (HRP peroxidase) reaction.
**Scheme 5.** Graphs of hERG inhibition for the pharmaceutical compounds **A-1, A-2.**
**Scheme 6.** ALT & AST levels in sera of all animals participating in the BLM-model.
   *(S + V): control, animals receiving saline instead of BLM and the vehicle of active pharmaceutical compounds; (B + V): animals receiving BLM and the vehicle of active pharmaceutical compounds; (B + A-1): animals receiving BLM and the pharmaceutical compound A-1; (B + A-2) animals receiving BLM and the pharmaceutical compound A-2.*
**Scheme 7.** Cell number (A) and total protein concentration (B) in the broncheoalveolar fluid of animals. Groups with statistically significant differences are denoted by "*".
   *(S + V): control, animals receiving saline instead of BLM and the vehicle of active pharmaceutical compounds; (B + V): animals receiving BLM and the vehicle of active pharmaceutical compounds; (B + A-1): animals receiving BLM and the pharmaceutical compound A-1; (B + A-2) animals receiving BLM and the pharmaceutical compound A-2.*
**Scheme 8.** Representative images of lung tissue sections from all groups following the BLM model. All images are at 40x magnification.
   *(S + V): control, animals receiving saline instead of BLM and the vehicle of active pharmaceutical compounds; (B + V): animals receiving BLM and the vehicle of active pharmaceutical compounds; (B + A-1): animals receiving BLM and the pharmaceutical compound A-1; (B + A-2) animals receiving BLM and the pharmaceutical compound A-2.*
**Scheme 9.** Fold change of mRNA levels of col1α1 and fibronectin in whole lung tissue samples after bleomycin model.
   *(S + V): control, animals receiving saline instead of BLM and the vehicle of active pharmaceutical compounds; (B + V): animals receiving BLM and the vehicle of active pharmaceutical compounds; (B + A-1): animals receiving BLM and the pharmaceutical compound A-1; (B + A-2) animals receiving BLM and the pharmaceutical compound A-2.*
**Scheme 10:** ATX inhibition attenuates inflammation and pulmonary edema post bleomycin (BLM) administration. a) Inflammatory cell numbers in bronchoalveolar lavage fluids (BALFs), as counted with a hematocytometer. b) Total protein concentration in BALFs, as determined with the Bradford assay. Statistical significance was assessed with one-way ANOVA; **/***/****denotes p < 0,01, 0,001, 0,0001 respectively; SAL-saline, BLM-bleomycin.
**Scheme 11:** ATX inhibition leads to the formation of fewer fibrotic lesions in murine fibrotic lungs. Representative images from lung sections of murine lungs, stained with H&E; SAL-saline, BLM-bleomycin.
**Scheme 12:** ATX inhibition improves mouse respiratory functions post-Bleomycin administration. Respiratory functions of mice were evaluated with the FlexiVent mechanical ventilator before their sacrifice. Representative markers are shown; IC-inspiratory capacity, Crs- mean respiratory system compliance, Ers- mean respiratory system elastance, H-mean tissue elastance, Cst-mean static lung compliance, A-mean total lung capacity, K-curvature of the upper portion of the deflation limp of the pressure volume (PV) curve. Statistical significance was assessed with one-way ANOVA; */**denotes p < 0,05, 0,01 respectively; SAL-saline, BLM-bleomycin.
**Scheme 13.** Synthetic route of the pharmaceutical compound **A-2.**
**Scheme 14.** Synthetic route of the pharmaceutical compound **A-3.**

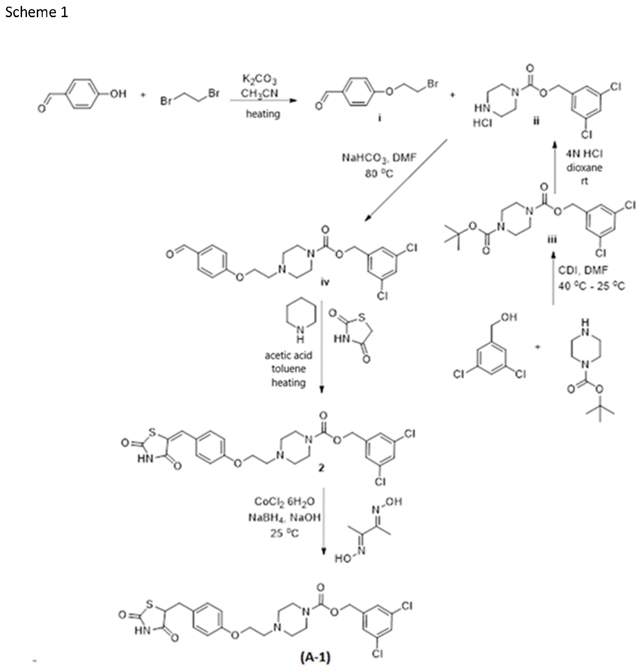

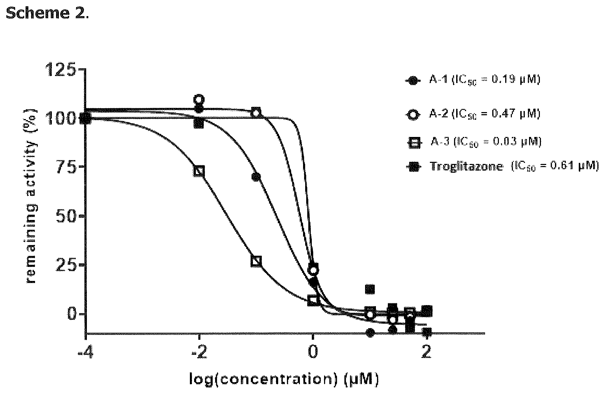

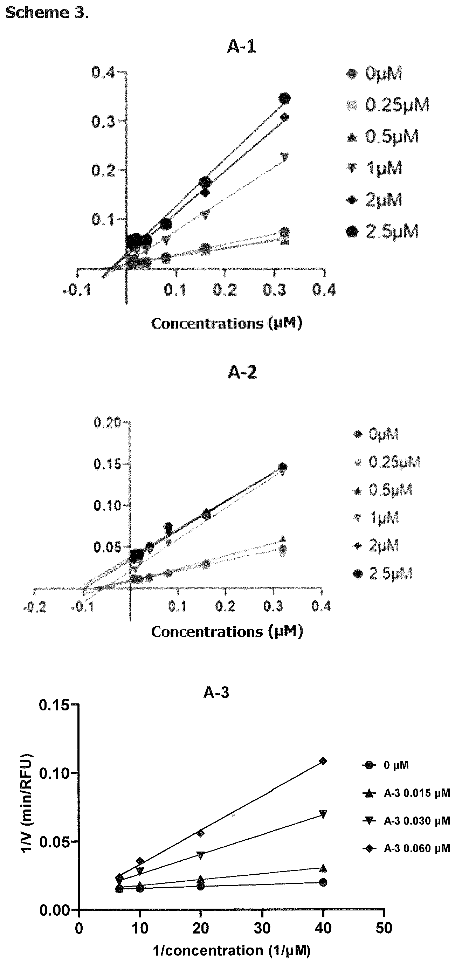

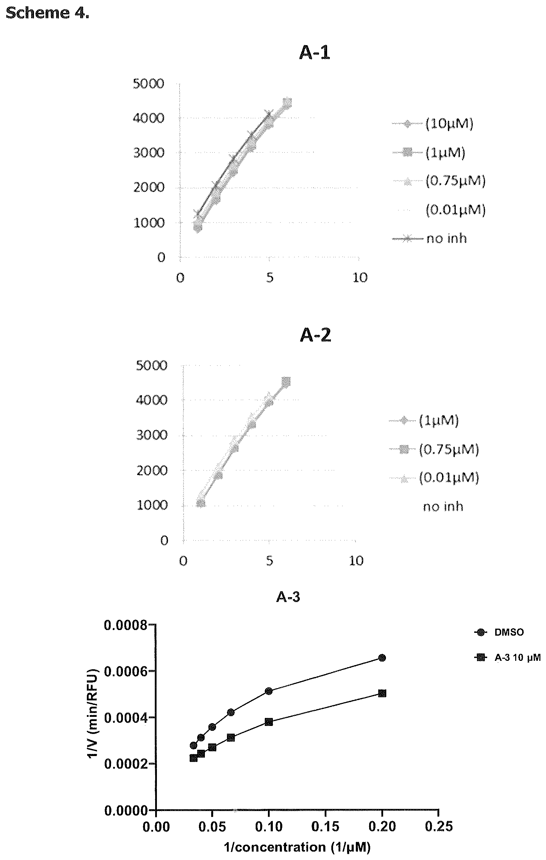

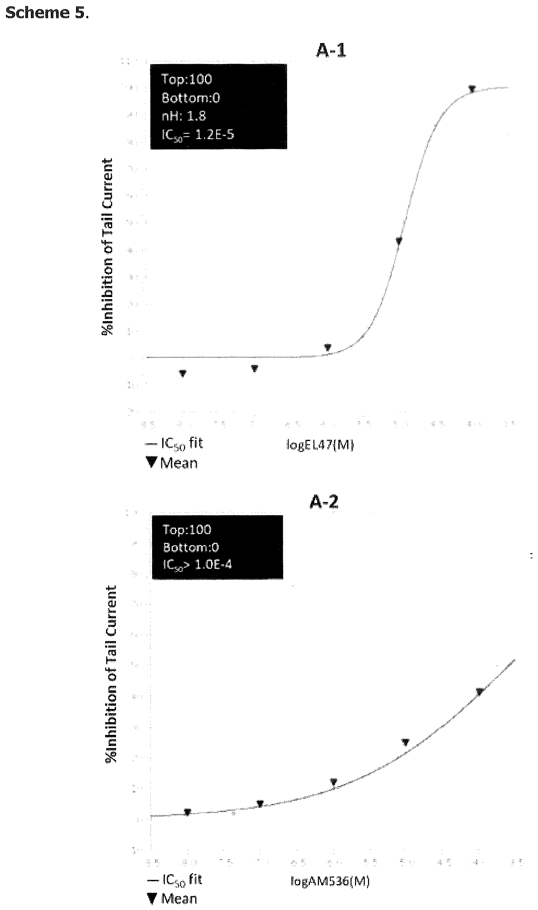

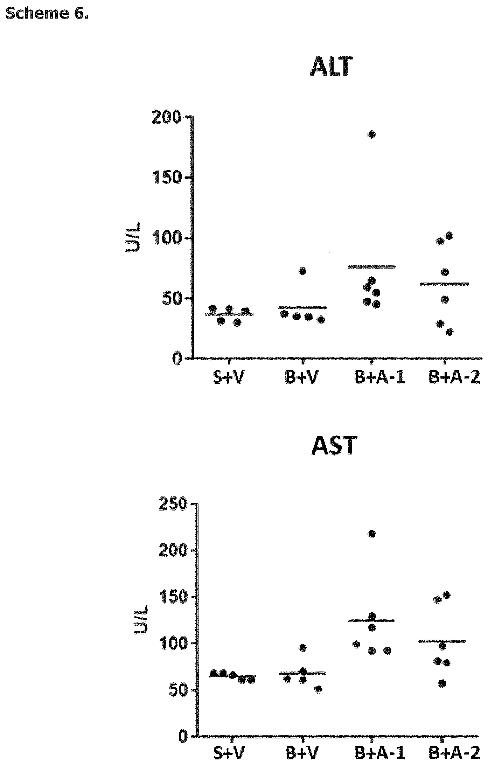

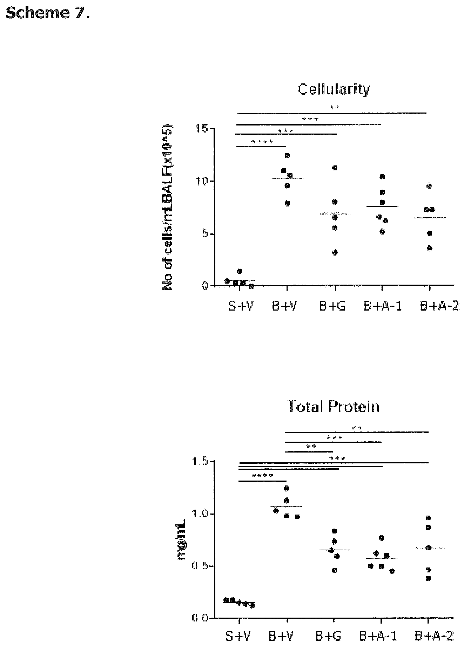

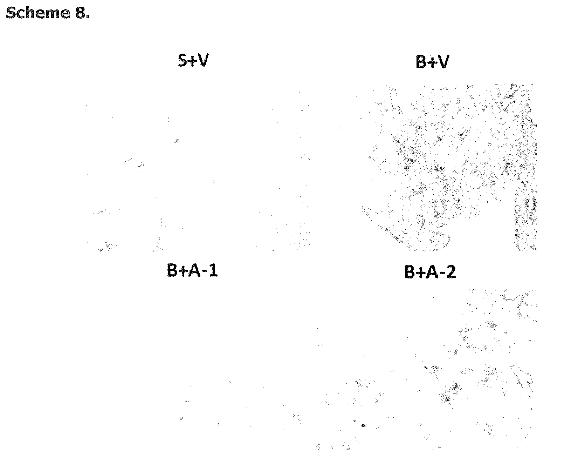

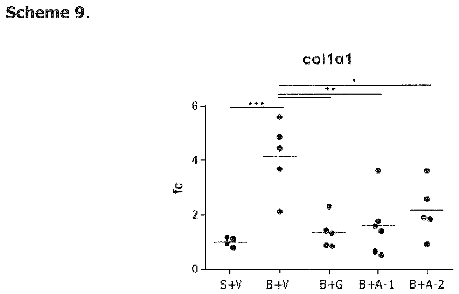

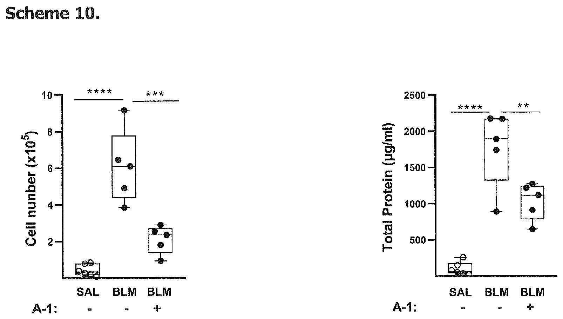

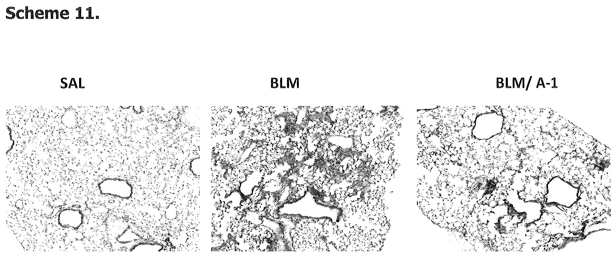

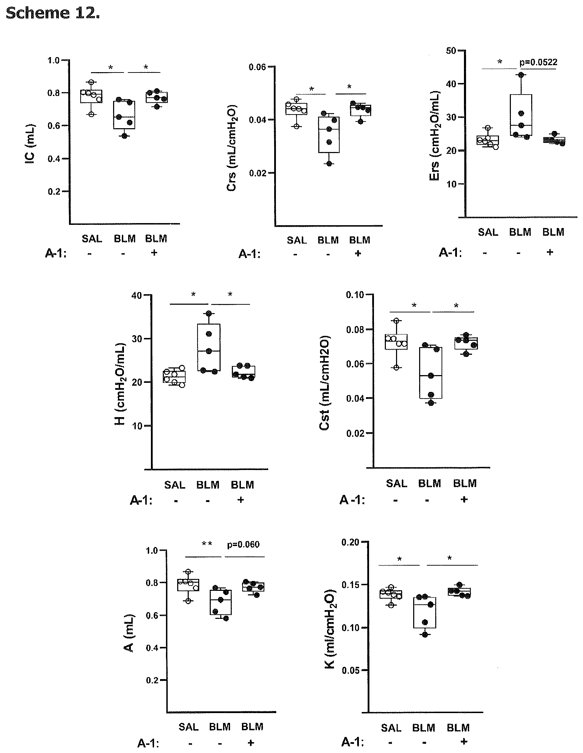

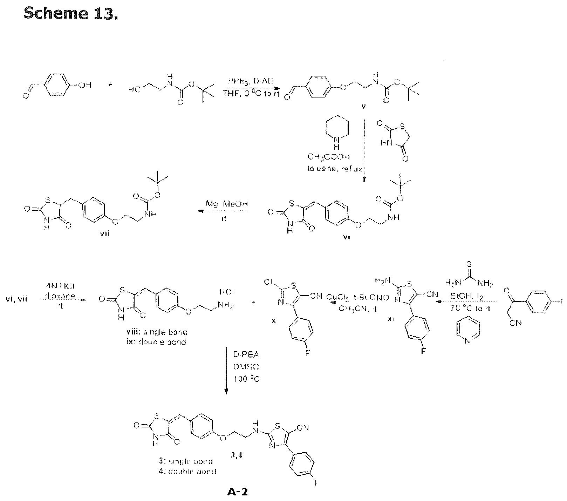

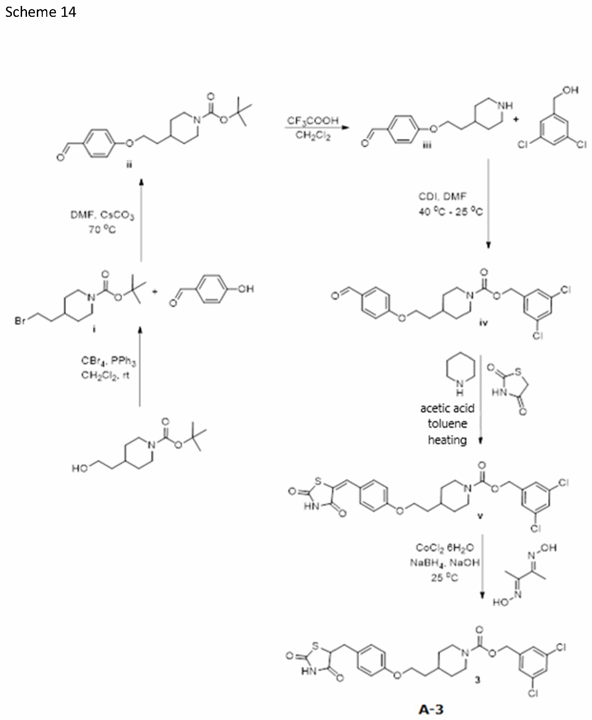

## Claims

1. A pharmaceutical compound or pharmaceutically acceptable salt thereof, for use in the prevention or the treatment of fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD due to simultaneous Autotaxin (ATX) inhibition and PPARγ agonism, having the chemical formula selected from the chemical formulas (A-1), (A-2) and (A-3)

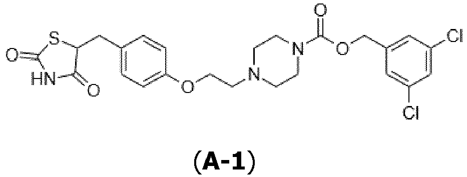

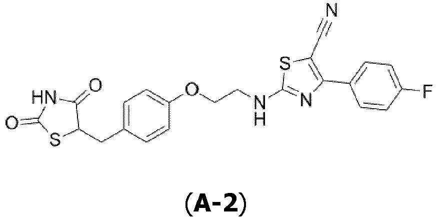

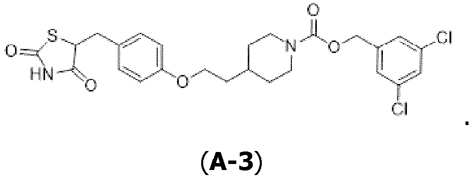

2. A pharmaceutical composition comprising a compound having the chemical formula selected from the chemical formulas (A-1), (A-2) and (A-3) 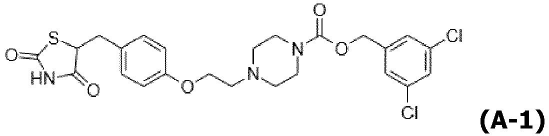 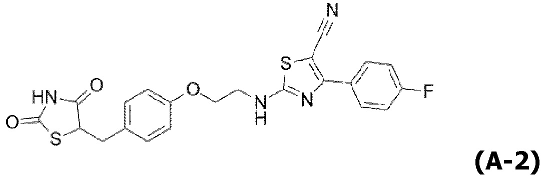 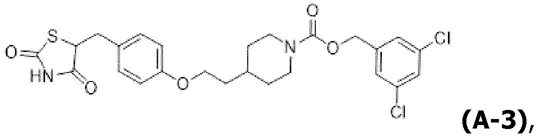 furthermore, comprising one or more pharmaceutically acceptable excipients, for use in the prevention or the treatment of fibro-proliferative diseases, in particular interstitial lung diseases (ILD) and/or liver diseases, such as all forms of hepatitis and/or non-alcoholic fatty liver disease (NAFLD) and/or non-alcoholic steatohepatitis (NASH) and/or cirrhosis, wherein said ILD being either primary disease, preferably idiopathic pulmonary fibrosis and/or sarcoidosis and/or interstitial pneumonias or said ILD being comorbid to autoimmune and/or inflammatory and/or metabolic diseases, preferably rheumatoid arthritis-ILD and/or scleroderma-ILD and/or myositis-ILD and/or diabetes-ILD and/or cardiovascular diseases-ILD.

3. The pharmaceutical composition for use according to claim 2, formulated with one or more excipients, the method comprising inhalational or intraperitoneal or oral or intranasal or subcutaneous or intravenous or topical administration to a subject in need thereof, preferably inhalational administration to a subject in need thereof.

## Patentansprüche

1. Pharmazeutische Verbindung oder ein pharmazeutisch annehmbares Salz davon zur Verwendung bei der Vorbeugung oder Behandlung von fibroproliferativen Erkrankungen, insbesondere interstitiellen Lungenerkrankungen (ILD) und/oder Lebererkrankungen, wie beispielsweise allen Formen der Hepatitis und/oder der nichtalkoholischen Fettlebererkrankung (NAFLD) und/oder der nichtalkoholischen Steatohepatitis (NASH) und/oder Leberzirrhose, wobei die ILD entweder als Primärerkrankung vorliegt, vorzugsweise als idiopathische Lungenfibrose und/oder Sarkoidose und/oder interstitielle Pneumonien, oder wobei die ILD als Komorbidität zu Autoimmun- und/oder Entzündungs- und/oder Stoffwechselerkrankungen auftritt, vorzugsweise als rheumatoide Arthritis-ILD und/oder Sklerodermie-ILD und/oder Myositis -ILD und/oder Diabetes-ILD und/oder kardiovaskuläre Erkrankungen-ILD, bedingt durch die gleichzeitige Hemmung von Autotaxin (ATX) und PPARγ-Agonismus, mit einer chemischen Formel, die aus den chemischen Formeln (A-1), (A-2) und (A-3) ausgewählt ist

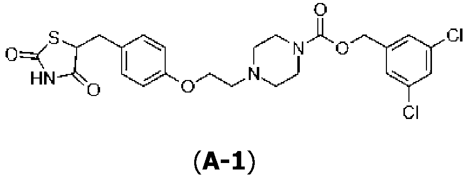

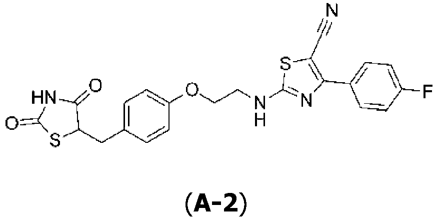

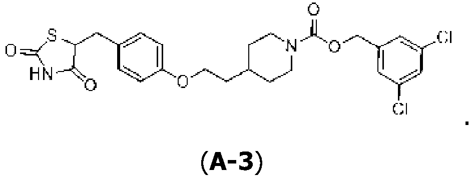

2. Pharmazeutische Zusammensetzung, umfassend eine Verbindung mit der chemischen Formel, ausgewählt aus den chemischen Formeln (A-1), (A-2) und (A-3) 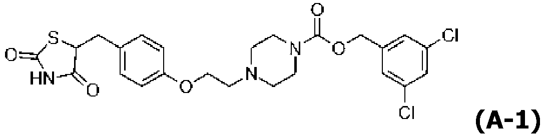 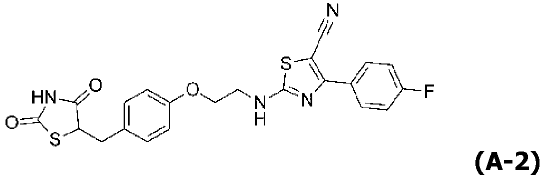 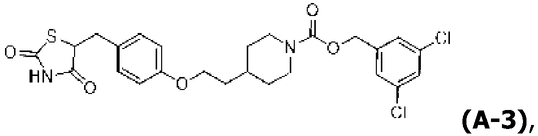 außerdem einen oder mehrere pharmazeutisch annehmbare Hilfsstoffe umfassend, zur Verwendung bei der Vorbeugung oder Behandlung von fibroproliferativen Erkrankungen, insbesondere interstitiellen Lungenerkrankungen (ILD) und/oder Lebererkrankungen, wie beispielsweise allen Formen der Hepatitis und/oder der nichtalkoholischen Fettlebererkrankung (NAFLD) und/oder der nichtalkoholischen Steatohepatitis (NASH) und/oder Leberzirrhose, wobei die ILD entweder als Primärerkrankung vorliegt, vorzugsweise als idiopathische Lungenfibrose und/oder Sarkoidose und/oder interstitielle Pneumonien, oder wobei die ILD als Komorbidität zu Autoimmun- und/oder Entzündungs- und/oder Stoffwechselerkrankungen auftritt, vorzugsweise als rheumatoide Arthritis-ILD und/oder Sklerodermie-ILD und/oder Myositis -ILD und/oder Diabetes-ILD und/oder kardiovaskuläre Erkrankungen-ILD.

3. Pharmazeutische Zusammensetzung zur Verwendung nach Anspruch 2, formuliert mit einem oder mehreren Hilfsstoffen, wobei das Verfahren die inhalative, intraperitoneale, orale, intranasale, subkutane, intravenöse oder topische Verabreichung an einen Patienten, der diese benötigt, umfasst, vorzugsweise die inhalative Verabreichung an einen Patienten, der diese benötigt.

## Revendications

1. Composé pharmaceutique ou sel pharmaceutiquement acceptable de celui-ci, pour une utilisation dans la prévention ou le traitement des maladies fibro-prolifératives, en particulier de maladies pulmonaires interstitielles (ILD) et/ou de maladies hépatiques, telles que toutes les formes d'hépatite et/ou de stéatose hépatique non alcoolique (NAFLD) et/ou de stéatohépatite non alcoolique (NASH) et/ou de cirrhose, dans laquelle ladite ILD est soit une maladie primitive, de préférence la fibrose pulmonaire idiopathique et/ou la sarcoïdose et/ou les pneumonies interstitielles, soit ladite ILD est une comorbidité associée à des maladies auto-immunes et/ou inflammatoires et/ou métaboliques, de préférence une polyarthrite rhumatoïde-ILD et/ou une sclérodermie-ILD et/ou une myosite-ILD et/ou un diabète-ILD et/ou des maladies cardiovasculaires-ILD, en raison de l'inhibition de l'autotaxine (ATX) et de l'agonisme du PPARγ simultanés, présentant une formule chimique choisie parmi les formules chimiques (A-1), (A-2) et (A-3)

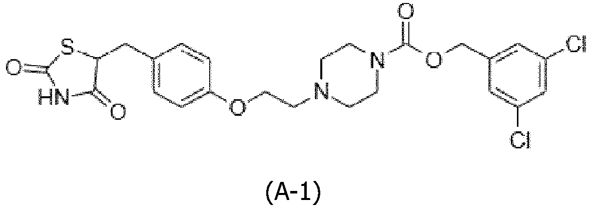

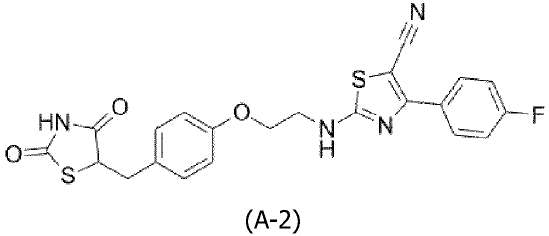

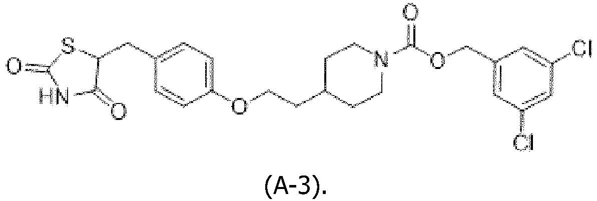

2. Composition pharmaceutique comprenant un composé présentant une formule chimique choisie parmi les formules (A-1), (A-2) et (A-3), 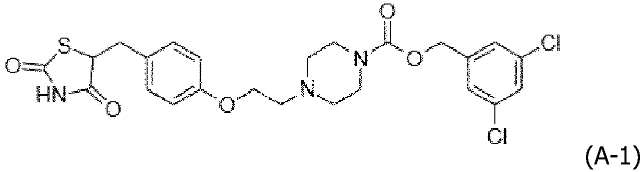 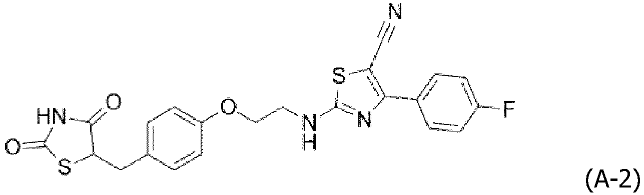 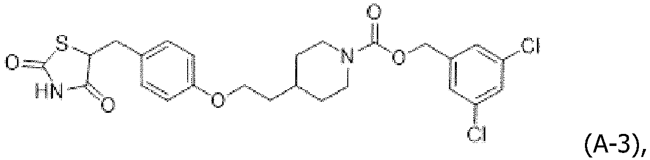 comprenant, en outre, un ou plusieurs excipients pharmaceutiquement acceptables, pour une utilisation dans la prévention ou le traitement de maladies fibro-prolifératives, en particulier de maladies pulmonaires interstitielles (ILD) et/ou de maladies hépatiques, telles que toutes les formes d'hépatite et/ou de stéatose hépatique non alcoolique (NAFLD) et/ou de stéatohépatite non alcoolique (NASH) et/ou de cirrhose, dans laquelle ladite ILD est soit une maladie primitive, de préférence la fibrose pulmonaire idiopathique et/ou la sarcoïdose et/ou les pneumonies interstitielles, soit ladite ILD est une comorbidité associée à des maladies auto-immunes et/ou inflammatoires et/ou métaboliques, de préférence une polyarthrite rhumatoïde-ILD et/ou une sclérodermie-ILD et/ou une myosite-ILD et/ou un diabète-ILD et/ou des maladies cardiovasculaires-ILD.

3. Composition pharmaceutique pour une utilisation selon la revendication 2, formulée avec un ou plusieurs excipients, la méthode comprenant une administration par voie inhalée ou intrapéritonéale ou orale ou intranasale ou sous-cutanée ou intraveineuse ou topique à un sujet en ayant besoin, de préférence une administration par voie inhalée à un sujet en ayant besoin.
